# EUROPÄISCHE PATENTANMELDUNG

(11) **EP 4 374 901 A1**
(43) Veröffentlichungstag der Anmeldung: **29.05.2024**
(21) Anmeldenummer: 22209706.5
(22) Anmeldetag: 25.11.2022
(51) Int. Cl.: A61M 16/08, A61M 16/00, A61M 16/20, A61M 16/22, A61M 16/10

(54) **BEATMUNGSGERÄT UND VERFAHREN ZUR WIEDERAUFBEREITUNG EINES AUSATEMGASES**

(71) Anmelder: Winkler, Bernd Erhard, 97286 Sommerhausen (DE)
(72) Erfinder: Winkler, Bernd Erhard, 97286 Sommerhausen (DE)
(74) Vertreter: Maikowski & Ninnemann Patentanwälte Partnerschaft mbB

(57) **Zusammenfassung**

Die Erfindung betrifft ein Beatmungsgerät zur Beatmung einer Person, aufweisend einen Strömungskreislauf für Atemgas mit: einem selbstinflatierenden Beatmungsbeutel, wobei der Beatmungsbeutel einen Patientenanschluss aufweist und wobei der Beatmungsbeutel weiter einen Auslass zur Einleitung eines Ausatemgases in den Strömungskreislauf und einen Einlass zum Ansaugen eines wiederaufbereiteten Ausatemgases aus dem Strömungskreislauf in den Beatmungsbeutel aufweist; mit einer Extraktionseinheit zum Entfernen von Kohlendioxid aus dem Ausatemgas; mit einer Frischgaszufuhr zur Zufuhr von Sauerstoff in den Strömungskreislauf; und mit einem Atemgasreservoir zur Zwischenspeicherung von im Strömungskreislauf befindlichem Atemgas. Die Erfindung betrifft ferner ein Verfahren zur Wiederaufbereitung von Ausatemgas in einem Strömungskreislauf eines solchen Beatmungsgeräts sowie eine Verwendung eines solchen Beatmungsgeräts zur Beatmung einer Person.

## Beschreibung

Die vorliegende Erfindung betrifft ein Beatmungsgerät zur Beatmung einer Person, wobei das Beatmungsgerät einen Strömungskreislauf für Atemgas aufweist, sowie ein Verfahren zur Wiederaufbereitung eines Ausatemgases in einem Strömungskreislauf eines Beatmungsgeräts.

Beatmungsgeräte kommen zum Einsatz, wenn aufgrund einer unzureichenden Eigenatmung oder einer schwerwiegenden Störung des Gasaustausches, wie beispielsweise bei einem Sauerstoffmangel oder einem Kohlendioxidüberschuss, eine maschinelle oder manuelle Beatmung einer Person notwendig wird. Dabei wird im Regelfall das von der Person ausgestoßene Atemgas, auch Ausatemgas genannt, an die Umgebung abgegeben. Da der Mensch lediglich ca. 4% des zugeführten Sauerstoffs verstoffwechseln kann, wird bei diesen sogenannten offenen Atemsystemen ein Großteil des zugeführten Sauerstoffs wieder ungenutzt an die Umgebung abgegeben. Das bedeutet, dass bei einer Beatmung beispielsweise mit 100% Sauerstoff, 96% des Sauerstoffs nicht für die Versorgung genutzt wird, sondern ungenutzt an die Umgebung abgegeben wird. Es gibt aber Situationen, in denen lebensrettender Sauerstoff nur in begrenztem Maße zur Verfügung steht und ein derartig verschwenderischer Umgang mit dem begrenzt vorhandenen Sauerstoff zu Versorgungsengpässen führt, die unter Umständen Leben kosten können. Insbesondere die Corona-Pandemie hat gezeigt, wie schnell lebensrettender Sauerstoff knapp werden und hierdurch eine lebensbedrohliche Situation entstehen kann.

Allgemein kann ein rascher Anstieg in der Zahl von zu beatmenden Personen, wie bei einer Pandemie, einem größeren Unfall oder einer Naturkatastrophe, zu einem Engpass in der Versorgung mit Sauerstoff führen. Aber auch in Luft- und Raumfahrt, bei der Schifffahrt auf hoher See und bei militärischen und humanitären Einsätzen sind die Sauerstoffvorräte begrenzt.

Aus dem Stand der Technik sind, insbesondere in der Anästhesie, auch geschlossene Atemsysteme bekannt, die das Ausatemgas wiederaufbereiten und das wiederaufbereitete Ausatemgas als Einatemgas zur Beatmung wiederverwenden.

Nachteilig bei diesen, aus dem Stand der Technik bekannten, geschlossenen Systemen ist, dass sowohl die Sauerstoffkonzentration als auch das Gasvolumen im System engmaschig überwacht und gegebenenfalls nachjustiert werden müssen. Dies macht einen weitgehend unbeobachteten Betrieb eines solchen geschlossenen Atemsystems mit einem hohen Anteil an wiederaufbereitetem Ausatemgas unmöglich. Geschlossene Atemsysteme kommen auch im Arbeitsschutz, beispielsweise bei der Rettung von Menschen in Gruben und Bergwerken, zum Einsatz. Die hier verwendeten Beatmungsgeräte setzen jedoch voraus, dass die zu beatmende Person noch zur Spontanatmung fähig ist.

Vor diesem Hintergrund ist es Aufgabe der vorliegenden Erfindung, ein Beatmungsgerät zur Verfügung zu stellen, das eine effizientere Nutzung des zur Verfügung stehenden Sauerstoffs erlaubt, und gleichzeitig auch weitgehend unbeobachtet zur Beatmung, auch bei nicht mehr erhaltener Spontanatmung, eingesetzt werden kann. Ferner ist es Aufgabe der vorliegenden Erfindung, ein Verfahren zur Wiederaufbereitung eines Ausatemgases zur Verfügung zu stellen.

Diese Aufgabe wird durch ein Beatmungsgerät zur Beatmung einer Person mit den Merkmalen des Anspruchs 1 sowie durch ein Verfahren zur Wiederaufbereitung von Ausatemgas in einem Strömungskreislauf eines Beatmungsgeräts mit den Merkmalen des Anspruchs 18, sowie einer Verwendung eines Beatmungsgeräts mit den Merkmalen des Anspruchs 28 gelöst.

Das Beatmungsgerät weist einen Strömungskreislauf für Atemgas auf. Im Sinne der vorliegenden Erfindung bezeichnet ein Atemgas ein bei der Beatmung einer Person mit dem Beatmungsgerät im Strömungskreislauf vorliegendes Gasgemisch. Dieses Gasgemisch kann beispielsweise das von der beatmeten Person ausgeatmete Ausatemgas umfassen und/oder andere Gase wie Sauerstoff, sauerstoffhaltige Gasgemische und/oder Umgebungsluft. Vorliegend wird der Begriff "Atemgas" allgemein für das im Strömungskreislauf des Beatmungsgeräts vorliegende Gasgemisch verwendet.

Das erfindungsgemäße Beatmungsgerät weist im Strömungskreislauf einen selbstinflatierenden Beatmungsbeutel, im Folgenden kurz als Beatmungsbeutel bezeichnet, auf. Der Beatmungsbeutel ist ein aus einem elastischen Material gefertigter Beutel oder Hohlkörper, dessen Volumen durch Kompression verkleinert werden kann. Der Beatmungsbeutel ist selbstinflatierend, das heißt, nach der Kompression erfolgt die Dekompression aufgrund der elastischen Rückstellkräfte des Beutels selbsttätig, ohne dass hierfür äußere Kräfte notwendig sind. Der erfindungsgemäße Beatmungsbeutel verfügt über einen Patientenanschluss zur Einleitung eines Ausatem- oder Expirationsgases in den Strömungskreislauf und zum Ausstoß eines Einatem- oder Inspriationsgases aus dem Strömungskreislauf. Dabei bezeichnet "Ausatemgas" das von einer zu beatmenden Person ausgeatmete Gas, "Einatemgas" das zur Einatmung zur Verfügung gestellte Gas. Über den Patientenanschluss kann das Beatmungsgerät mit der Lunge einer zu beatmenden Person strömungsverbunden werden. Vorliegend wird das durch den Patientenanschluss in den Strömungskreislauf eingeleitete Ausatemgas im Strömungskreislauf wiederaufbereitet und das so wiederaufbereitete Ausatemgas als Einatemgas wiederverwertet. Dies ermöglicht eine effiziente Nutzung von Sauerstoffvorräten. Dies ist insbesondere in Situationen wichtig, in denen der zur Verfügung stehende Sauerstoff begrenzt ist.

Der Patientenanschluss weist in einer Variante ein Bauteil zur Trennung von Ausatemgas und Einatemgas, beispielsweise ein sogenanntes Y- oder T-Stück, auf. Ein Beispiel für ein Y- oder T-Stück ist ein sogenanntes Patientenventil. Das Patientenventil ist beispielsweise ein Zwei-Wege-Ventil und kann als Entenschnabel- oder Lippenventil oder als Diskus- oder Tellerventil ausgeführt sein. Über das Y- oder T-Stück, insbesondere das Patientenventil, wird Ausatemgas in den Strömungskreislauf geleitet und das wiederaufbereitete Ausatemgas als Einatemgas aus dem Strömungskreislauf abgegeben.

Der Beatmungsbeutel weist einen Auslass zur Einleitung von Ausatemgas in den Strömungskreislauf auf. Dieser wird im Folgenden kurz als Ausatemgas-Auslass bezeichnet. Dieser ist beispielsweise mit dem Patientenanschluss strömungsverbunden. Ausatemgas gelangt so von der Lunge über den Patientenanschluss durch den Ausatemgas-Auslass in den Strömungskreislauf. Der Patientenanschluss ist in einer Variante ferner mit einem zweiten Auslass des Beatmungsbeutels strömungsverbunden, durch den bei Kompression des Beutels das in diesem befindliche wiederaufbereitete Ausatemgas aus dem Beutel ausgestoßen werden kann. Dieser zweite Auslass wird im Folgenden kurz als Einatemgas-Auslass bezeichnet. Das abgegebene wiederaufbereitete Ausatemgas passiert in dieser Variante das Y- oder T-Stück und kann so über den Patientenanschluss einer zu beatmenden Person als Einatemgas zugeführt werden. Dabei erfolgt der Ausstoß aus dem Beatmungsbeutel durch Kompression desselben, das Ansaugen durch die selbsttätige Dekompression des Beutels.

Der Beatmungsbeutel weist außerdem einen Einlass zum Ansaugen eines wiederaufbereiteten Ausatemgases aus dem Strömungskreislauf in den Beatmungsbeutel auf. Beispielsweise ist der Einlass mit einem Einlassventil ausgestattet, das ein Rückströmen von wiederaufbereitetem Ausatemgas aus dem Beatmungsbeutel durch den Einlass in den Strömungskreislauf verhindert.

Ferner ist im Strömungskreislauf eine Extraktionseinheit angeordnet. In der Extraktionseinheit wird das durch den Ausatemgas-Auslass in den Strömungskreislauf eingebrachte Ausatemgas von Kohlendioxid befreit. Die Extraktionseinheit ist beispielsweise als Atemkalkbehälter ausgeführt. Dieser enthält zum Beispiel eine alkalische Verbindung zur Aufnahme von Kohlendioxid, wie Natrium- und/oder Calciumhydroxid. Das in dem Ausatemgas befindliche Kohlendioxid reagiert mit den Hydroxid-Ionen zu Wasser und Natrium- und/oder Calciumcarbonat. Die bei dieser exothermen Reaktion entstehende Wärme kann zur Erwärmung des Atemgases genutzt werden, das entstehende Wasser zur Anfeuchtung desselben. In einer Variante ist die als Atemkalkbehälter ausgeführte Extraktionseinheit mit einem pH-Indikator ausgestattet. Mittels des pH-Indikators, beispielsweise Farb-pH-lndikators, lässt sich einfach und kostengünstig eine nachlassende Alkalität und somit nachlassende Wirksamkeit des Atemkalks signalisieren und ablesen. Alternativ oder ergänzend können in der Extraktionseinheit Temperatursensoren zur Detektion einer Erschöpfung des Atemkalks angebracht sein. Im zeitlichen Verlauf reagieren zunächst die dem Eingang der Extraktionseinheit nahegelegenen Kalkschichten mit dem Kohlendioxid und erst nach und nach die dem Ausgang nähergelegenen Schichten. Ein Temperaturanstieg im Sinne einer beginnenden exothermen Reaktion in den dem Ausgang der Extraktionseinheit nahegelegenen Kalkschichten kann somit eine bevorstehende Erschöpfung des Atemkalks anzeigen. Die Extraktionseinheit verfügt beispielsweise über einen Eingang zur Zuführung von Ausatemgas und über einen Ausgang zur Abführung von gereinigtem Ausatemgas. Vorliegend wird das in der Extraktionseinheit von Kohlendioxid befreite Ausatemgas als "gereinigtes Ausatemgas" bezeichnet. Dabei bezieht sich eine Entfernung von Kohlendioxid sowohl auf eine vollständige als auch auf eine teilweise Entfernung von Kohlendioxid aus dem Atemgas. Der Eingang ist beispielsweise mit dem Ausatemgas-Auslass strömungsverbunden. Zwischen dem Ausatemgas-Auslass und dem Eingang der Extraktionseinheit können weitere Komponenten, beispielsweise Ventile, angeordnet sein.

Der Strömungskreislauf weist ferner eine Frischgaszufuhr auf. Diese ist beispielsweise zwischen der Extraktionseinheit und dem Einlass des Beatmungsbeutels angeordnet.

Mittels der Frischgaszufuhr wird, beispielsweise dem aus dem Ausgang der Extraktionseinheit abgeführten, gereinigten, Ausatemgas Sauerstoff zugeführt. Dabei umfasst die Zufuhr von Sauerstoff sowohl die Zufuhr von reinem Sauerstoff als auch die Zufuhr von sauerstoffhaltigen, insbesondere sauerstoffreichen, Gasgemischen. Durch die Zufuhr des Sauerstoffs, beispielsweise zum gereinigten Ausatemgas, wird das bei der Beatmung in der zu beatmenden Person verstoffwechselte Sauerstoffvolumen kompensiert. Das so erzeugte wiederaufbereitete Ausatemgas kann dann als Einatemgas mittels des Beatmungsbeutels über den Patientenanschluss appliziert werden.

Außerdem weist der Strömungskreislauf ein Atemgasreservoir zur Zwischenspeicherung von im Strömungskreislauf befindlichem Atemgas auf. Dabei umfasst das Atemgas das Ausatemgas und/oder das gereinigte Ausatemgas und/oder das wiederaufbereitete Ausatemgas. Das Atemgasreservoir dient als Gegenlunge. Das Atemgasreservoir ist beispielsweise als elastischer Beutel oder als Zylinder mit Kolben oder als Zylinder mit dehnbarer Membran ausgeführt. In einer Variante ist das Atemgasreservoir zwischen der Extraktionseinheit und der Frischgaszufuhr angeordnet.

In einer Variante weist das Beatmungsgerät mindestens ein Verbindungselement, beispielsweise ein Verbindungsrohr und/oder einen Verbindungsschlauch, zur Verbindung von Komponenten des Strömungskreislaufs auf. Die Gesamtheit der Verbindungselemente kann als alleiniges oder zusätzliches Atemgasreservoir dienen. Die Verbindungselemente stellen insbesondere eine gasdichte Verbindung zwischen den entsprechenden Komponenten her. Alternativ können Komponenten auch unmittelbar nebeneinander angeordnet und direkt miteinander verbunden sein, so dass kein Verbindungselement benötigt wird.

Grundsätzlich können Extraktionseinheit, Atemgasreservoir und Frischgaszufuhr in beliebiger Reihenfolge im Strömungskreislauf angeordnet sein. Jede Reihenfolge hat ihre spezifischen Vor- und Nachteile. Beispielsweise muss bei einer Anordnung der Frischgaszufuhr vor der Extraktionseinheit bei der Einatmung zusätzlich der Strömungswiderstand der Extraktionseinheit, insbesondere eines darin angeordneten Atemkalks überwunden werden.

In einer weiteren Variante ist ein Ventil zur Erzeugung eines positiven endexspiratorischen Drucks ("positive end-exspiratory pressure, PEEP"), ein sogenanntes PEEP-Ventil, im Strömungskreislauf angeordnet. Beispielsweise ist das PEEP-Ventil zwischen dem Ausatemgas-Auslass des Beatmungsbeutels und dem Eingang der Extraktionseinheit angeordnet oder in den Ausatemgas-Auslass integriert. Der positive endexspiratorische Druck verhindert ein weitgehendes Kollabieren der Lunge, sorgt für eine homogenere Dehnung der einzelnen Lungenareale und verbessert in der Lunge den Gasaustausch mit dem Blut. Das PEEP-Ventil kann auch als Rückschlagventil fungieren und die Einatmung von Ausatemgas, d.h. nicht wiederaufbereitetem Ausatemgas, bei Spontanatmung verhindern, wie sie sonst durch den Ausatemgas-Auslass und durch den Patientenanschluss bei alleiniger Verwendung des Patientenventils möglich wäre. Das PEEP-Ventil weist beispielsweise einen Schaltdruck zwischen 0 und 30 mbar, insbesondere zwischen 0 und 20 mbar auf. In einer Variante liegt der Schaltdruck des PEEP-Ventils zwischen 5 und 18 mbar.

Alternativ kann ein positiver end-exspiratorischer Druck auch durch eine Verringerung eines Strömungsquerschnitts der Extraktionseinheit in Strömungsrichtung erreicht werden. Denn weist die Extraktionseinheit einen sich entlang der Strömungsrichtung verringernden, beispielsweise verjüngenden, Querschnitt auf, wird hierdurch ein Strömungswiderstand erzeugt, der wiederum zur Erzeugung einer positiven Druckdifferenz zwischen Patientenanschluss bzw. der Lunge einer daran angeschlossenen Person und dem Atemgasreservoir genutzt werden kann. In dieser Variante ist die Extraktionseinheit zwischen Ausatemgas-Auslass und Atemgasreservoir angeordnet. Ist die Extraktionseinheit beispielsweise als Atemkalkbehälter ausgeführt, so wird eine Verringerung des Strömungsquerschnitts bereits aufgrund des Atemkalks erreicht.

In einer weiteren Variante ist ein Überdruckventil zum Ablassen von Atemgas aus dem Beatmungsbeutel und zur Zuführung des abgelassenen Atemgases an die Extraktionseinheit zwischen dem Patientenanschluss und dem Eingang der Extraktionseinheit angeordnet. Dieses wird im Folgenden als Atemwegs-Überdruckventil bezeichnet. Das Atemwegs-Überdruckventil kann mit konstantem oder mit einstellbarem Schaltdruck ausgeführt sein. Dabei definiert der Schaltdruck den maximalen Atemwegsdruck. Beispielsweise liegt der Schaltdruck des Atemwegs-Überdruckventils über dem Schaltdruck des PEEP-Ventils, insbesondere zwischen 30 und 70 mbar. In einer Variante liegt der Schaltdruck zwischen 50 und 60 mbar. Zusätzlich kann ein optisches und/oder akustisches Warnelement vorgesehen sein, welches das Überschreiten des Schaltdrucks signalisiert. Das Warnelement kann elektronisch und/oder pneumatisch-mechanisch ausgeführt sein. Ein Warnelement kann beispielsweise eine oder mehrere Pfeifen oder ein oder mehrere Flatterbänder oder ein oder mehrere Rotoren aufweisen, die von dem über das Atemwegs-Überdruckventil austretenden Atemgasstrom betätigt werden.

Das Atemgasreservoir kann in einer Variante ferner ein Überdruckventil aufweisen, über das überschüssiges Atemgas aus dem Strömungskreislauf abgelassen werden kann, wenn ein kritischer Füllzustand bzw. ein kritischer Fülldruck überschritten wird. Auch das Überdruckventil, im Folgenden auch Atemgasreservoir-Überdruckventil genannt, kann als Ventil mit einem konstanten Schaltdruck oder als Ventil mit einem einstellbaren Schaltdruck ausgeführt sein. Dabei bezeichnet Schaltdruck den Druck im Atemgasreservoir, bei dessen Überschreiten das Atemgas abgelassen wird. Beispielsweise liegt der Schaltdruck des Atemgasreservoir-Überdruckventils zwischen 0 und 20 mbar, zum Beispiel zwischen 0 und 10 mbar, insbesondere zwischen 1 und 5 mbar. Das abgelassene Atemgas wird in die Umgebung abgelassen. Das abgelassene Atemgas kann dabei beispielsweise über einen Blasen-Diffusor geleitet werden, um die Größe der entstehenden Gasblasen zu verringern. Dies ist insbesondere bei militärischen Einsätzen unter Wasser relevant, bei denen eine Enttarnung vermieden werden sollte. Alternativ oder zusätzlich kann das abgelassene Atemgas über einen Sauerstoffreduktor geleitet werden. Durch die chemische Reduktion des reaktiven Sauerstoffs aus dem abgelassenen Atemgas mittels eines geeigneten Reduktionsmittels kann ein sicheres Ablassen auch in einer reaktiven Umgebung sichergestellt werden. Auch die Bildung von Gasblasen unter Wasser kann so unterbunden oder verringert werden.

In einer Variante ist die Frischgaszufuhr zwischen dem Atemgasreservoir und dem Einlass des Beatmungsbeutels angeordnet. Insbesondere erfolgt die Frischgaszufuhr stromabwärts hinter dem Atemgasreservoir.

In einer weiteren Variante ist ein Noteinlassventil zum Einlass von Umgebungsluft in den Strömungskreislauf, insbesondere zwischen der Frischgaszufuhr und dem Einlass des Beatmungsbeutels, angeordnet. Das Noteinlassventil stellt bei einem Ausfall der Frischgaszufuhr sicher, dass zumindest Umgebungsluft in den Strömungskreislauf gelangen und sich der Beatmungsbeutel trotz fehlender Frischgaszufuhr füllen kann. Das Noteinlassventil stellt somit bei einem Ausfall der Frischgaszufuhr eine Notfallbeatmung mit Umgebungsluft sicher.

Ferner ist in einer Variante ein Filter zur Entfernung von toxischen Gasen und/oder toxischen Partikeln aus dem Strömungskreislauf, insbesondere zwischen der Frischgaszufuhr und dem Einlass des Beatmungsbeutels, angeordnet. Toxische Gase und/oder toxische Partikel umfassen atomare, biologische und chemische Noxen. Die toxischen Partikel können hierbei als Feststoffe und/oder Flüssigkeiten vorliegen. Die Entfernung kann vollständig oder teilweise sein und beispielsweise durch Binden der toxischen Gase und/oder Partikel im Filter erfolgen. Durch das Entfernen der toxischen Gase und/oder Partikel aus dem Strömungskreislauf wird auch eine Anreicherung der toxischen Gase und/oder Partikel bei einer Beatmung im Atemgas verhindert. Der Filter ist beispielsweise ein ABC-Filter und weist in einer Variante Aktivkohle und/oder ein Molekularsieb auf.

In einer Variante weist die Frischgaszufuhr eine Sauerstoffquelle und/oder einen Anschluss für eine externe Sauerstoffquelle auf. Über die Sauerstoffquelle und/oder den Anschluss für eine externe Sauerstoffquelle kann Sauerstoff, d.h. reiner Sauerstoff oder ein sauerstoffhaltiges Gasgemisch, in den Strömungskreislauf eingeleitet werden. Eine Sauerstoffquelle ist beispielsweise ein Druckgasbehälter, z.B. eine Sauerstoffflasche. Alternativ oder ergänzend kann die Sauerstoffquelle als Kryo-Sauerstoff und/oder als Sauerstoff-Konzentrator zur Verfügung gestellt werden. Die Sauerstoffquelle kann in einer alternativen Variante Sauerstoff und gegebenenfalls weitere Gase wie beispielsweise Stickstoff aus der Umgebungsluft abtrennen. Dies kann beispielsweise in der Sauerstoffquelle mittels des Linde-Verfahrens erfolgen. Die hierfür nötige Abkühlung kann insbesondere unter Ausnutzung des Joule-Thomson-Effekts erfolgen. In einer weiteren Variante kann die Sauerstoffquelle Sauerstoff mittels Druckwechsel-Adsorption ("pressure swing adsorption, PSA") aus der Umgebungsluft abtrennen. Hierbei erfolgt eine Gleichgewichtsadsorption oder eine Trennung aufgrund der Molekularsiebwirkung mit Hilfe poröser Adsorber-Materialien, wie z. B. Zeolithe und/oder Aktivkohle. In einer weiteren Variante kann die Sauerstoffquelle Sauerstoff und Stickstoff aus der Umgebungsluft mittels mindestens einer semipermeablen Membran abtrennen. Auch ein chemischer Sauerstoffgenerator kann als Sauerstoffquelle dienen. Hier wird Sauerstoff mittels einer chemischen Reaktion aus entsprechenden Ausgangssubstanzen gewonnen. Die chemische Erzeugung des Sauerstoffs erfolgt beispielsweise aus Chloraten, z.B. Natriumchlorat. In einer alternativen Ausführungsform erfolgt die chemische Sauerstofferzeugung beispielsweise aus Hyperoxiden, z.B. Natrium-Hyperoxid.

In einer Variante weist die Frischgaszufuhr eine bedarfsgesteuerte Dosierung und/oder eine Konstantdosierung auf. Bei der Konstantdosierung wird dem Strömungskreislauf ein konstanter Volumenstrom an Sauerstoff, d.h. reinem Sauerstoff oder einem sauerstoffhaltigen Gasgemisch, zugeführt. Bei der bedarfsgesteuerten Dosierung variiert das Volumen an zugeführtem Sauerstoff entsprechend dem Sauerstoff-Bedarf der zu beatmenden Person. Beispielsweise entspricht das Volumen des zugeführten Sauerstoffs dem Volumen des im Körper der zu beatmenden Person verstoffwechselten Sauerstoffs. Die bedarfsgesteuerte Dosierung kann anstelle der Konstantdosierung oder zusätzlich zu dieser eingesetzt werden. Sie wird elektronisch oder mechanisch-pneumatisch gesteuert. Die bedarfsgesteuerte Dosierung ist in der einfachsten Ausführung ein bedarfsgesteuertes Gas-Einlassventil, auch Demandventil genannt. Das Demandventil öffnet bei Anliegen einer negativen Druckdifferenz beim Ansaugen von wiederaufbereitetem Ausatemgas in den Beatmungsbeutel und lässt Gas in den Strömungskreislauf, bis keine Druckdifferenz mehr anliegt und Druckgleichheit erreicht ist. Dies stellt sicher, dass bei der Dekompression des Beatmungsbeutels zunächst das Atemgasreservoir entleert wird und dann, bei nicht-ausreichendem Gasvolumen im Strömungskreislauf, das Demandventil betätigt wird. Somit kann eine Ersetzung des verstoffwechselten Sauerstoffvolumens im Ausatemgas umgesetzt werden. Der Schaltdruck des Demandventils genügt in einer Ausführungsform der EN250 für Tauch-Atemregler. Beispielsweise liegt der Schaltdruck des Demandventils unter 25 mbar, insbesondere 10 mbar, in einer Variante unter 5 mbar, sogar unter 2 mbar. Als Demandventil kann aus beispielsweise auch ein Tauch-Atemregler eingesetzt werden. Der Schaltdruck des Demandventils ist beispielsweise so gewählt, dass zunächst das Atemgasreservoir möglichst vollständig entleert wird, bevor eine Zudosierung von Frischgas erfolgt. Die Kombination von bedarfsgesteuerter Dosierung mit Konstantdosierung maximiert die Patientensicherheit, wie im Bezug auf die speziellen Ausführungsformen noch ausgeführt wird. Alternativ oder zusätzlich zu der bedarfsgesteuerten Dosierung mittels Demandventil ist in einer Variante eine elektrische bedarfsgesteuerte Dosierung vorgesehen.

In einer Variante sind bedarfsgesteuerte Dosierung und Konstantdosierung in einem Bauteil kombiniert. Dies wird beispielsweise durch eine im Demandventil verbaute Injektordüse erreicht oder auch durch eine entsprechende Vorspannung eines Kipphebels eines Demandventils. So wird bereits ohne Erzeugung eines Unterdrucks ein Frischgasfluss erreicht, welcher bei Erzeugung eines Unterdrucks bedarfsgerecht erhöht wird.

In einer Variante weist die Konstantdosierung eine Vorrichtung zur Deaktivierung der Konstantdosierung auf. Dies ermöglicht eine situationsabhängige Umstellung auf eine ausschließlich bedarfsgesteuerte Dosierung. Dies kann beispielsweise in reaktiver, insbesondere explosiver Umgebung oder bei militärischen Unterwassereinsätzen relevant sein und sollte unter vorsichtiger Abwägung der mit einer alleinigen bedarfsgesteuerten Dosierung verbundenen Risiken erfolgen.

Ferner ist in einer Variante ein Spontanatmungs-Bypass mit Rückschlagventil, insbesondere zwischen Frischgaszufuhr und einem Auslass des Strömungskreislaufes, parallel zum Beatmungsbeutel angeordnet, wobei der Auslass geeignet ist, mit der Lunge einer an das Beatmungsgerät angeschlossenen Person verbunden zu werden. Insbesondere weist das Rückschlagventil des Spontanatmungs-Bypasses, im Folgenden Spontanatmungs-Bypass-Ventil genannt, einen geringeren Atemwiderstand auf als die im parallelen, den Beatmungsbeutel enthaltenden Ast des Strömungskreislaufs angeordneten Ventile. Das heißt, der Atemwiderstand des Spontanatmungs-Bypass-Ventils ist geringer als der Atemwiderstand der Kombination von Einlassventil und Patientenventil des Beatmungsbeutels. Hierdurch wird die Einatemarbeit einer selbsttätig atmenden Person reduziert. Die zu beatmende Person muss zur Spontanatmung weniger Kraft und Energie aufwenden, um einen eigenständigen Atemhub anzufordern. Das Rückschlagventil weist beispielsweise einen Schaltdruck unter 10 mbar, insbesondere unter 5 mbar, in einer Variante unter 2 mbar auf.

In einer weiteren Variante ist im Strömungskreislauf eine Sauerstoffmessvorrichtung zur Bestimmung der Sauerstoffkonzentration im wiederaufbereiteten Ausatemgas angeordnet. Hierdurch lässt sich die Sauerstoffkonzentration im wiederaufbereiteten Ausatemgas überwachen, und, insbesondere in Verbindung mit der Frischgaszufuhr, steuern und/oder regeln. Die Bestimmung ist qualitativ und/oder quantitativ. Die quantitative Sauerstoffmessvorrichtung verfügt beispielsweise über einen akustischen und/oder optischen Alarm zur Signalisierung einer Abweichung der tatsächlichen Sauerstoffkonzentration im wiederaufbereiteten Atemgas von einem Sollwert. Hierdurch wird eine unbemerkte Abweichung der tatsächlichen Sauerstoffkonzentration von einem Sollwert verhindert. Die über die Frischgaszufuhr zugeführte Sauerstoffmenge kann in Abhängigkeit von der gemessenen Sauerstoffkonzentration eingestellt werden. Frischgaszufuhr und Sauerstoffmessvorrichtung sind zu diesem Zweck beispielsweise miteinander und/oder mit einer Steuer- und/oder Regeleinheit, bevorzugt einer Recheneinheit, verbunden.

Alternativ oder zusätzlich ist im Strömungskreislauf eine Kohlendioxidmessvorrichtung zur Bestimmung der Kohlendioxidkonzentration im Ausatemgas und/oder im wiederaufbereiteten Ausatemgas angeordnet. Diese ist beispielsweise zwischen dem Ausatemgang-Auslass des Beatmungsbeutels und der Extraktionseinheit angeordnet.

Hierdurch lässt sich die Kohlendioxidkonzentration im Ausatemgas und/oder im wiederaufbereiteten Ausatemgas überwachen. Insbesondere die Kohlendioxidkonzentration im Ausatemgas erlaubt direkten Rückschluss auf die Kohlendioxidkonzentration im Blut der zu beatmenden Person. Hierdurch sind Rückschlüsse auf die Beatmungsqualität und -quantität möglich. Eine übermäßige Beatmung (Hyperventilation) kann damit ebenso erkannt und folglich auch verhindert werden wie eine unzureichende Beatmung (Hypoventilation). Durch Messung der Kohlendioxidkonzentration im wiederaufbereiteten Ausatemgas kann verhindert werden, dass unzureichend wiederaufbereitetes, kohlendioxidhaltiges Ausatemgas appliziert wird. Die Bestimmung ist qualitativ und/oder quantitativ. Die quantitative Kohlendioxidmessvorrichtung verfügt beispielsweise über einen akustischen und/oder optischen Alarm zur Signalisierung einer Abweichung der tatsächlichen Kohlendioxidkonzentration im wiederaufbereiteten Atemgas von einem Sollwert. Hierdurch wird eine unbemerkte Abweichung der tatsächlichen Kohlendioxidkonzentration von einem Sollwert verhindert. Bereits durch eine qualitative Kohlendioxidmessung kann eine Fehlfunktion der Extraktionseinheit, beispielsweise eine Erschöpfung des Atemkalks, erkannt werden und entsprechende Gegenmaßnahmen getroffen werden.

In einer weiteren Variante weist das Beatmungsgerät einen automatischen Antrieb zur Kompression des Beatmungsbeutels auf. Hierdurch entfällt die Notwendigkeit einer manuellen Beatmung, insbesondere das manuelle Auspressen des Beatmungsbeutels. Die Beatmung kann somit auch in Abwesenheit von Personal erfolgen. Der automatische Antrieb kann eine Vorrichtung zur maschinellen Auspressung des Beatmungsbeutels umfassen. Solche Vorrichtungen umfassen beispielsweise einen oder mehrere Exzenter, Zugbänder, Kolben oder Linearmotoren. Auch eine Vorrichtung zum pneumatischen oder hydraulischen Auspressen mittels eines Kolbens oder mittels eines gasgefüllten bzw. mittels eines flüssigkeitsgefüllten Verdrängungssackes kann als automatischer Antrieb dienen. Alternativ kann ein automatischer Antrieb durch eine parallel zum Beatmungsbeutel im Strömungskreislauf angeordnete Pumpe umgesetzt sein. Als Pumpe kann beispielsweise eine Kolbenpumpe, Rotationspumpe, Flügelpumpe, Turbine und/oder Membranpumpe zum Einsatz kommen. Insbesondere ist eine Recheneinheit zur Steuerung und/oder Regelung des automatischen Antriebs vorgesehen. Beispielsweise sind Druck- und/oder Gasflusssensoren im Strömungskreislauf angeordnet, um die selbsttätige Atemarbeit der zu beatmenden Person bei Spontanatmung zu detektieren und/oder zu quantifizieren und/oder eine zeitliche Synchronisation von maschinellen Atemhüben mit selbsttätiger Atemarbeit zu erzielen.

Die Druck- und/oder Gasflusssensoren sind bevorzugt patientennah positioniert, beispielsweise dem Patientenanschluss vorgelagert, so dass Ausatemgas zuerst die Sensoren und dann den Patientenanschluss passiert. Alternativ oder zusätzlich sind die Sensoren der Pumpe nachgelagert, so dass wiederaufbereitetes Ausatemgas zuerst die Pumpe und dann die Sensoren passiert.

In einer Variante weist das Beatmungsgerät eine Recheneinheit zur Steuerung und/oder Regelung des automatischen Antriebs auf. Durch Verbindung mit der Sauerstoff- und/ oder Kohlendioxidmessvorrichtung kann optional zusätzlich eine Steuerung und/oder Regelung einer Beatmungsfrequenz und/oder eines Atemzugvolumens auf Basis der von der bzw. den Messvorrichtungen ermittelten Messergebnisse erfolgen.

In einer Variante weist das Beatmungsgerät ein Befestigungselement zur Befestigung des Beatmungsgeräts an einer Patiententrage auf. Hierdurch wird insbesondere eine Verwendung des Beatmungsgeräts unter Wasser ermöglicht. Das Beatmungsgerät lässt sich mit Hilfe des Befestigungselements relativ zu einer zu beatmenden Person, beispielsweise auf einer Patiententrage, fixieren.

Ein weiterer Gegenstand ist eine Beatmungseinheit, wobei die Beatmungseinheit mindestens ein erstes und ein zweites Beatmungsgerät mit den Merkmalen der Ansprüche 1 bis 17 aufweist. Das erste und zweite Beatmungsgerät befinden sich in unmittelbarer räumlicher Nähe zueinander. In dieser Variante wird ein einziges Demandventil für die bedarfsgesteuerte Dosierung der Frischgaszufuhr verwendet. Die Einspeisung des Frischgases in das erste und zweite Beatmungsgerät erfolgt über Verbindungselemente. Ein Rückströmen von Atemgas aus dem ersten und zweiten Beatmungsgerät in Richtung des gemeinsamen Demandventils wird durch Rückschlagventile verhindert. Zum Schutz vor gegenseitiger Kontamination mit Krankheitserregern können entsprechende Mikroben-Filter eingesetzt werden. Die Beatmungseinheit weist in einer Variante mehrere Beatmungsgeräte auf, wobei die mehreren Beatmungsgeräte ein Demandventil teilen.

Ein weiterer Gegenstand ist ein Verfahren zur Wiederaufbereitung von Ausatemgas in einem Strömungskreislauf eines Beatmungsgeräts, insbesondere eines Beatmungsgeräts nach einem der Ansprüche 1 bis 17, mit den Schritten: Einleitung von Ausatemgas in einen in dem Strömungskreislauf angeordneten selbstinflatierenden Beatmungsbeutel über einen Patientenanschluss des Beatmungsbeutels durch einen Auslass des Beatmungsbeutels in den Strömungskreislauf; Entfernen von Kohlendioxid aus dem Ausatemgas mittels einer im Strömungskreislauf angeordneten Extraktionseinheit, insbesondere zur Erzeugung eines gereinigten Ausatemgases; Zwischenspeicherung von im Strömungskreislauf befindlichem Atemgas in einem Atemgas-Reservoir des Beatmungsgeräts; Zufuhr von Sauerstoff in den Strömungskreislauf mittels einer Frischgaszufuhr, insbesondere Zufuhr von Sauerstoff zum gereinigten Ausatemgas zur Erzeugung eines wiederaufbereiteten Ausatemgases; Ansaugen eines wiederaufbereiteten Ausatemgases durch einen Einlass des Beatmungsbeutels in den Beatmungsbeutel durch Dekompression des Beatmungsbeutels; und Ausstoß des wiederaufbereiteten Ausatemgases aus dem Strömungskreislauf durch den Patientenanschluss des Beatmungsbeutels durch Kompression des Beatmungsbeutels. Das erfindungsgemäße Verfahren ermöglicht die Wiederaufbereitung von Ausatemgas in einem Beatmungsgerät und somit den effizienten Einsatz von Sauerstoff in einem Beatmungsgerät.

Das Verfahren setzt die im Zusammenhang mit dem Beatmungsgerät beschriebenen Vorteile auf Verfahrensebene um.

In einer Variante wird während der Einleitung des Ausatemgases in den Strömungskreislauf ein positiver exspiratorischer Druck (PEEP) aufrechterhalten.

In einer weiteren Variante wird bei Überschreitung eines definierten Drucks des Ausatemgases in dem Beatmungsbeutel Ausatemgas aus dem Beatmungsbeutel mittels eines Atemwegs-Überdruckventils abgelassen und der Extraktionseinheit zugeführt.

Alternativ oder zusätzlich wird Atemgas mittels eines Atemgasreservoir-Überdruckventils aus dem Strömungskreislauf abgelassen, wenn ein Schaltdruck des Atemgasreservoir-Überdruckventils überschritten wird. Das Ablassen erfolgt an die Umgebung, beispielsweise über einen Blasen-Diffusor und/oder einen Sauerstoffreduktor.

In einer Variante erfolgt die Zufuhr von Sauerstoff über eine Frischgaszufuhr mit Konstantdosierung und/oder über eine Frischgaszufuhr mit bedarfsgesteuerter Dosierung. Beispielsweise wird die Konstantdosierung deaktiviert, so dass ausschließlich eine bedarfsgesteuerte Dosierung der Frischgaszufuhr erfolgt.

In einer Variante wird das wiederaufbereitete Ausatemgas aus dem Strömungskreislauf zusätzlich durch einen Spontanatmungs-Bypass aus dem Strömungskreislauf abgegeben, wobei der Spontanatmungs-Bypass ein parallel zum Beatmungsbeutel angeordnetes Rückschlagventil aufweist. Die Abgabe über den Spontanatmungs-Bypass benötigt bei Spontanatmung des Patienten weniger Kraft und Energie als der Ausstoß über den Beatmungsbeutel.

In einer Variante ist vorgesehen, dass mittels eines im Strömungskreislauf angeordneten Filters, beispielsweise mittels eines ABC-Filters, toxische Gase und/oder toxische Partikel aus dem Strömungskreislauf entfernt werden.

Ferner wird in einer Variante eine Sauerstoffkonzentration des wiederaufbereiteten Ausatemgases gemessen und, beispielsweise, die Frischgaszufuhr basierend auf den so erhaltenen Messwerten mittels einer Steuer- und Regeleinheit, bevorzugt einer Recheneinheit, gesteuert und/oder geregelt. Alternativ oder zusätzlich wird die Kohlendioxidkonzentration des Ausatemgases und/oder des wiederaufbereiteten Ausatemgases gemessen. Beispielsweise kann mittels der gemessenen Kohlendioxidkonzentration im Ausatemgas die Beatmungsqualität und -quantität bestimmt werden. Die Messung der Kohlendioxidkonzentration im wiederaufbereiteten Ausatemgas ermöglicht eine Überwachung der Reinigung des Ausatemgases, insbesondere in der Extraktionseinheit. Die Frischgaszufuhr wird beispielsweise basierend auf den erhaltenen Messwerten mittels einer Steuer- und Regeleinheit, z.B. einer Recheneinheit, gesteuert und/oder geregelt.

In einer Variante wird der Beatmungsbeutel durch einen automatischen Antrieb komprimiert. Anstelle des manuellen Auspressens des Beatmungsbeutels tritt somit beispielsweise entweder das maschinelle Auspressen mittels einer geeigneten Vorrichtung oder das Pumpen von wiederaufbereitetem Ausatemgas mittels einer parallel zum Beatmungsbeutel im Strömungskreislauf angeordneten Pumpe.

Ein weiterer Gegenstand ist eine Verwendung eines Beatmungsgeräts nach einem der Ansprüche 1 bis 17 zur Beatmung einer Person.

In einer Variante umfasst die Verwendung die Fixierung des Beatmungsgeräts relativ zu einer zu beatmenden Person, beispielsweise mittels eines Befestigungselements auf einer Patiententrage. In dieser Variante ist die zu beatmende Person ebenfalls an der Patiententrage befestigt.

In einer Variante umfasst die Verwendung das Verkippen von relativ zueinander fixiertem Beatmungsgerät und zu beatmender Person zwischen einer ersten Position und einer zweiten Position. Beispielsweise ist das Beatmungsgerät in der ersten Position oberhalb, d.h. in Schwerkraftrichtung oberhalb, der zu beatmenden Person angeordnet. Beispielsweise ist das Beatmungsgerät in der zweiten Position unterhalb, d.h. in Schwerkraftrichtung unterhalb, der zu beatmenden Person angeordnet. Insbesondere bei einer Verwendung im bzw. unter Wasser führt ein in unterschiedlichen Höhen herrschender unterschiedlicher hydrostatischer Druck zu einem Einströmen bzw. Ausströmen von Atemgas in das und aus dem Beatmungsgerät, insbesondere in den und aus dem Beatmungsbeutel. Hierdurch wird ein manuelles oder maschinelles Auspressen des Beatmungsbeutels überflüssig.

Weitere Einzelheiten der vorliegend beschriebenen Erfindung sollen anhand eines Ausführungsbeispiels und entsprechender Figuren näher erläutert werden. Es zeigen:
- Figur 1: eine erste Ausführungsform des Beatmungsgeräts sowie eine erste Ausführungsform des erfindungsgemäßen Verfahrens;
- Figur 2: das Beatmungsgerät in einer zweiten Ausführungsform sowie eine zweite Ausführungsform des erfindungsgemäßen Verfahrens;
- Figur 3: das Beatmungsgerät in einer dritten Ausführungsform sowie eine dritte Ausführungsform des erfindungsgemäßen Verfahrens;
- Figur 4: das Beatmungsgerät in einer vierten Ausführungsform sowie eine vierte Ausführungsform des erfindungsgemäßen Verfahrens;
- Figur 5a: eine Verwendung eines Beatmungsgeräts in einer ersten Variante; und
- Figur 5b: eine Verwendung eines Beatmungsgeräts in einer zweiten Variante.

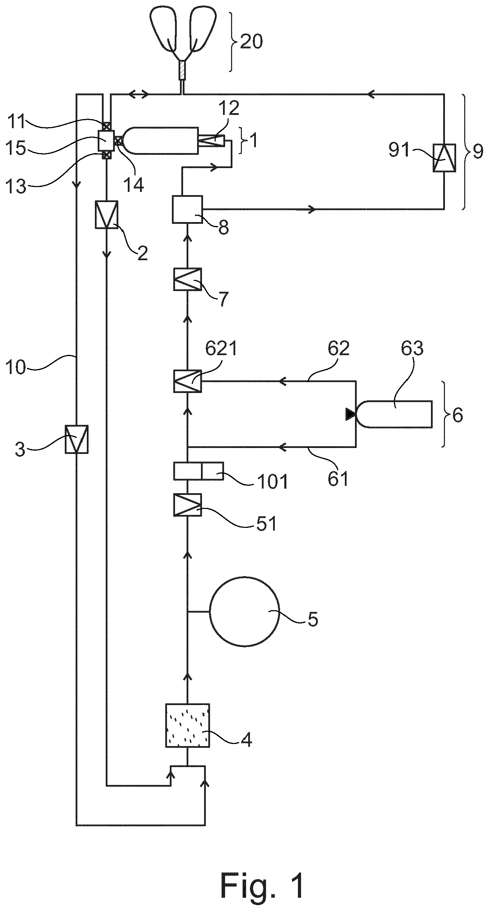

**Figur** 1 zeigt ein Beatmungsgerät 30 mit einem Strömungskreislauf, der mit einer Lunge 20 einer zu beatmenden Person strömungsverbunden ist. Im Strömungskreislauf ist ein Beatmungsbeutel 1 angeordnet. Der Beatmungsbeutel 1 ist ein elastischer Beutel, der durch Kompression und Dekompression in dem Beutel 1 befindliches Atemgas ausstoßen bzw. ansaugen kann. Der Beatmungsbeutel 1 weist einen Patientenanschluss 11 auf. Über diesen kann eine Strömungsverbindung zwischen der Lunge 20 der zu beatmenden Person und Beatmungsgerät 30 hergestellt werden. Über den Patientenanschluss 11 gelangt von der zu beatmenden Person ausgestoßenes Ausatemgas über einen Ausatemgas-Auslass 13 des Beatmungsbeutels 1 in den Strömungskreislauf des Beatmungsgeräts 30. Das Ausatemgas wird im Strömungskreislauf wiederaufbereitet. Durch Ansaugen des wiederaufbereiteten Ausatemgases aus dem Strömungskreislauf durch einen Einlass 12 des Beatmungsbeutel 1 gelangt das wiederaufbereitete Ausatemgas in den Beutel 1. Der Beatmungsbeutel 1 weist beispielsweise weiter einen Einatemgas-Auslass 14 auf, über den durch Kompression des Beutels 2 das wiederaufbereitete Ausatemgas dem Patientenanschluss 11 zugeleitet werden kann und hierdurch der zu beatmenden Person appliziert werden kann.

Der Einlass 12 weist beispielsweise ein Einlassventil auf, um zu verhindern, dass bei der Kompression das in dem Beatmungsbeutel 1 befindliche wiederaufbereitete Ausatemgas durch den Einlass 12 in den Strömungskreislauf zurückfließt. Die Trennung von Einatemgas und Ausatemgas im Beatmungsbeutel 1 erfolgt beispielsweise über ein zwischen Patientenanschluss 11, Ausatemgas-Auslass 13 und Einatemgas-Auslass 14 angeordnetes Zwei-Wege-Ventil 15.

Das Ausatemgas wird einer Extraktionseinheit 4 zugeleitet. In der Extraktionseinheit 4 wird Kohlendioxid aus dem Ausatemgas teilweise oder vollständig entfernt. Der Extraktionseinheit 4 wird somit kohlendioxidhaltiges Ausatemgas über einen Eingang zugeleitet. Durch einen Ausgang der Extraktionseinheit 4 wird gereinigtes Ausatemgas in den Strömungskreislauf gegeben. Die Extraktionseinheit 4 weist beispielsweise einen Atemkalkbehälter mit Atemkalk auf. Der Atemkalk reagiert mit dem in dem Ausatemgas befindlichen Kohlendioxid und entfernt dieses so teilweise oder vollständig aus dem Ausatemgas. Dabei entstehende Wärme kann zur Erwärmung des gereinigten Ausatemgases verwendet werden. Als Reaktionsprodukt anfallendes Wasser kann zur Befeuchtung des gereinigten Ausatemgases genutzt werden. Eine mögliche Erschöpfung des Atemkalks kann durch ein entsprechendes Warnelement, beispielsweise einen pH-Farbindikator und/oder Temperatursensoren angezeigt werden. Die Extraktionseinheit 4 ist beispielsweise zwischen Ausatemgas-Auslass 13 und dem Einlass 12 angeordnet. In einer Variante ist ein PEEP-Ventil 2 zwischen Ausatemgas-Auslass 13 und Extraktionseinheit 4 angeordnet. Dies stellt einen positiven endexspiratorischen Druck bei der Beatmung zur Verfügung. Das PEEP-Ventil 2 hat beispielsweise einen Schaltdruck zwischen 0 und 30 mbar, insbesondere zwischen 0 und 20 mbar, in der dargestellten Variante von 5 bis 18 mbar. Alternativ kann auch ein sich verringernder Strömungsquerschnitt der Extraktionseinheit 4 genutzt werden, um einen Strömungswiderstand zu erzeugen und so einen positiven endexspiratorischen Druck zur Verfügung zu stellen. Beispielsweise reduziert der Atemkalk in einer als Atemkalkbehälter ausgeführten Extraktionseinheit 4 den Strömungsquerschnitt und kann so einen positiven endexspiratorischen Druck sichern, ohne dass ein zusätzliches Bauteil hierfür notwendig ist. In einer Ausführungsform ohne PEEP-Ventil 2 kann im Strömungsverlauf hinter dem Ausatemgas-Auslass 13 oder in dem Ausatemgas-Auslass 13 ein Rückschlagventil integriert sein, um bei Spontanatmung der zu beatmenden Person in bestimmten Situationen ein ungewolltes Ansaugen von Ausatemgas durch das Zwei-Wege-Ventil 15 entgegen der Soll-Strömungsrichtung zu verhindern

Zur Sicherung vor Überdrücken ist zum Schutz der Lunge 20 vor zu hohen Atemwegsdrücken und zum Schutz vor einer damit einhergehenden Schädigung der Lunge 20 ein Atemwegs-Überdruckventil 3 zwischen Patientenanschluss 11 und Extraktionseinheit 4 angeordnet. Ein optisches und/oder akustisches Warnelement kann vorgesehen sein, um auf ein Ansprechen des Ventils 3 bzw. die damit einhergehenden zu hohen Atemwegsdrücke aufmerksam zu machen. Der Schaltdruck des Atemwegs-Überdruckventils 3 beträgt beispielsweise zwischen 30 und 70 mbar, in der dargestellten Variante zwischen 50 und 60 mbar.

Ferner ist im Strömungskreislauf ein Atemgasreservoir 5 vorgesehen. Das Atemgasreservoir 5 dient als Gegenlunge. Hier wird das Atemgas, insbesondere nach dem Ausatmen, gespeichert, bis es erneut mit einem Atemhub der zu beatmenden Person zugeführt werden kann. Das Atemgasreservoir 5 ist vorliegend als separate Einheit, beispielsweise als Beutel, als Zylinder mit Kolben oder als Zylinder mit einer dehnbaren Membran ausgeführt. Alternativ oder zusätzlich erfolgt die Speicherung des Atemgases in Verbindungselementen 10, mit denen einzelne Komponenten des Strömungskreislaufs miteinander strömungsverbunden sind. Verbindungselemente 10 sind beispielsweise Verbindungsschläuche mit einer gewissen Elastizität oder Verbindungsrohre von ausreichendem Volumen. Komponenten des Strömungskreislaufs, die nicht durch Verbindungselemente 10 miteinander strömungsverbunden sind, sind nebeneinander angeordnet, so dass eine direkte Verbindung der Komponenten möglich ist.

Das Atemgasreservoir 5 weist ebenfalls ein Überdruckventil, das Atemgasreservoir-Überdruckventil 51 auf. Erreicht das Volumen des Atemgasreservoirs 5, insbesondere bei Ausführung des Atemgasreservoirs 5 als separate Einheit wie in der Figur 1 dargestellt, einen kritischen Füllzustand, d.h. liegt am Atemgasreservoir-Überdruckventil 51 ein entsprechender kritischer Fülldruck an, öffnet das Ventil 51. Im dargestellten Beispiel liegt der Fülldruck beispielsweise zwischen 0 und 20 mbar, in einer Variante zwischen 0 und 10 mbar, ins der dargestellten Variante zwischen 1 und 5 mbar. Überschüssiges Atemgas wird an die Umgebung abgegeben. Dies kann, je nach Situation bzw. Einsatzgebiet des Beatmungsgeräts 30, über einen Blasen-Diffusor und/oder einen Sauerstoffreduktor erfolgen. Hierdurch wird die Größe und/oder Menge der austretenden Gase reduziert, was beispielsweise die Wahrscheinlichkeit einer Enttarnung bei militärischen Einsätzen reduziert.

Im Strömungskreislauf, beispielsweise stromabwärts des Atemgasreservoirs 5, ist eine Frischgaszufuhr 6 angeordnet. Mittels der Frischgaszufuhr wird dem gereinigten Ausatemgas Sauerstoff beigemischt. Da während eines Atemzugs nur ca. 4% des im Einatemgas enthaltenen Sauerstoffs verstoffwechselt werden, ist auch nur eine entsprechend geringe Menge Sauerstoff hinzuzufügen. Hierdurch wird ein effizienter Einsatz des vorhandenen Sauerstoffs ermöglicht. Der Sauerstoff kann als reiner Sauerstoff oder als sauerstoffhaltiges, insbesondere sauerstoffreiches, Gasgemisch zugeführt werden. Hierzu kann das Beatmungsgerät 30, wie in der dargestellten Ausführungsform, selbst über eine Sauerstoffquelle 63 verfügen. Alternativ kann als Teil des Beatmungsgeräts 30 ein Anschluss für eine externe Sauerstoffquelle vorgesehen sein. Als Sauerstoffquelle 63 dient beispielsweise ein Druckgasbehälter, d.h. eine Sauerstoffflasche. Die Frischgaszufuhr 6 verfügt in der vorliegenden Ausführungsform sowohl über eine Konstantdosierung 61 als auch über eine bedarfsgesteuerte Dosierung 62. Die bedarfsgesteuerte Dosierung 62 verfügt über ein Demandventil 621, das eine dem Bedarf angepasste Zufuhr von Sauerstoff umsetzt. Der Schaltdruck des Demandventils 621 liegt beispielsweise unter 25 mbar, insbesondere unter 10 mbar, im vorliegenden Beispiel unter 5 mbar. Der Schaltdruck kann aber auch unter 2 mbar liegen. Hinter der Frischgaszufuhr 6 liegt im Strömungskreislauf somit wiederaufbereitetes Ausatemgas vor, das als Einatemgas zur Beatmung einer Person eingesetzt werden kann.

Die Kombination von bedarfsgesteuerter Dosierung 62 mit Konstantdosierung 61 maximiert die Patientensicherheit. Durch die Konstantdosierung 61 wird dem Strömungskreislauf ein konstanter Fluss an Sauerstoff zugeführt. Zusätzlich wird in dem Fall, in dem die im Atemgasreservoir 6 vorhandene und die mittels Konstantdosierung 61 zugeführte Atemgasmenge nicht ausreicht, um das Atemhubvolumen bereitzustellen, die bedarfsgesteuerte Dosierung 62 aktiviert und zusätzlicher Sauerstoff zugeführt. Der zusätzliche Einsatz der bedarfsgesteuerten Dosierung 62 ist insbesondere relevant, wenn der über die Konstantdosierung 61 zur Verfügung gestellte Sauerstofffluss unzureichend ist. Ohne bedarfsgesteuerte Dosierung 62 wäre hier entweder das Atemhubvolumen unzureichend oder, bei ausgleichender Zumischung von Umgebungsluft, die Sauerstoffkonzentration unzureichend. Beides wird durch die bedarfsgesteuerte Dosierung 62 ausgeglichen. Aber auch durch Beimischung anderer Gase, insbesondere von Stickstoff, im Strömungskreislauf, kann die Kombination beider Dosierungen wichtig sein. Dies ist besonders bei einem Eindringen von Stickstoff relevant, da dieser sich im Strömungskreislauf bei der Beatmung akkumuliert. Dringt also auch nur eine geringe Menge Stickstoff in den Strömungskreislauf ein, beispielsweise aufgrund einer undichten Stelle im Strömungskreislauf, akkumuliert sich dieser. Die Stickstoffkonzentration steigt, während die Sauerstoffkonzentration entsprechend sinkt. Da der Stickstoff jedoch nicht, wie das Kohlendioxid, mittels einer Extraktionseinheit 4 aus dem Ausatemgas eliminiert wird, wird das Stickstoffvolumen auch nicht reduziert, so dass das Demandventil 621 nicht ausgelöst wird. In diesem Fall stellt die Konstantdosierung 61 die ausreichende Sauerstoffzufuhr sicher. Die Konstantdosierung 61 weist in einer Ausführungsform eine Vorrichtung zur Deaktivierung der Konstantdosierung 61 (nicht dargestellt) auf. Mittels dieser Vorrichtung lässt sich die Konstantdosierung 61 deaktivieren, d.h. ausschalten. Dies sollte unter sorgfältiger Nutzen-Risiko-Abwägung geschehen. Eine Deaktivierung der Konstantdosierung 61 kann beispielsweis beim Einsatz in einer reaktiven, insbesondere explosiven Umgebung angezeigt sein, in der ein durch die Konstantdosierung möglicherweise erzeugter Sauerstoffüberschuss zur Freisetzung von Sauerstoff in die reaktive Umgebung führen würde oder aber bei militärischen Unterwassereinsätzen, bei denen freigesetzte Gasblasen zur Enttarnung führen können.

Im Strömungskreislauf ist ferner ein Noteinlassventil 7, beispielsweise stromabwärts der Frischgaszufuhr 6, vorgesehen. Dies stellt bei einem Ausfall der Frischgaszufuhr 6 sicher, dass sich der selbstinflatierende Beatmungsbeutel 1 dekomprimieren, d.h. wieder ausdehnen, kann. Durch das Noteinlassventil 7 wird Umgebungsluft in den Strömungskreislauf eingelassen, so dass der Beatmungsbeutel 1 sich zumindest mit Umgebungsluft füllen und wenigstens eine Beatmung mit einem Gemisch aus gereinigtem Ausatemgas und Umgebungsluft erfolgen kann. Das Noteinlassventil 7 weist hierzu beispielsweise einen definierten Öffnungsdruck auf. Der negative Öffnungsdruck des Noteinlassventils 7 ist insbesondere kleiner, d.h. dem Betrag nach größer, als der negative Öffnungsdruck des Demandventils 621 der Frischgaszufuhr 6. Das heißt, bei leerem Atemgasreservoir 5 öffnet zunächst das Demandventil 621 und erst bei Ausfall desselben und bei Ausfall der entsprechenden Frischgaszufuhr 6 öffnet das Noteinlassventil 7. In einer Ausgestaltung ist das Noteinlassventil 7 mit dem Atemgasreservoir-Überdruckventil 51 konstruktiv in einem Bauteil kombiniert. Das Noteinlassventil 7 kann ebenfalls eine Vorrichtung zur Deaktivierung aufweisen, so dass ein Auslösen des Noteinlassventils 7 für bestimmte Einsatzzwecke unterbunden werden kann. Dies ist immer dann der Fall, wenn eine Beatmung mit Umgebungsluft nicht möglich oder nicht erwünscht ist, beispielsweise bei Einsätzen unter Wasser oder in toxischer und/oder reaktiver Umgebung.

Im Strömungskreislauf kann weiter ein Filter für toxische Gase und/oder Partikel, insbesondere ein ABC-Filter 8, vorgesehen sein. Hierdurch wird dem bei einem Einsatz in toxischer Umgebung bestehende Risiko begegnet, dass toxisches Gas in den Strömungskreislauf gelangt. Dies ist besonders dann relevant, wenn der Atemweg der zu beatmenden Person nicht durch Intubation gesichert ist, beispielsweise wenn die Beatmung über eine Gesichtsmaske erfolgt. Toxische Gase und/oder toxische Partikel können sich im Strömungskreislauf anreichern. Der ABC-Filter 8 entfernt diese toxischen Gase und/oder toxischen Partikel vollständig oder teilweise aus dem Strömungskreislauf, beispielsweise indem er sie bindet, und verhindert damit eine Anreicherung und/oder Beatmung mit giftigen Gasen. Der ABC-Filter 8 weist beispielsweise Aktivkohle und/oder ein Molekularsieb auf.

In einer Ausführungsform ist ein Spontanatmungs-Bypass 9 ist im Strömungskreislauf parallel zum Beatmungsbeutel 1 angeordnet. Durch den Spontanatmungs-Bypass 9 kann ebenfalls wiederaufbereitetes Ausatemgas aus dem Strömungskreislauf abgegeben und einer zu beatmenden Person zugeführt werden. Hierzu ist ein entsprechendes Bypass-Ventil oder Spontanatmungs-Bypass-Ventil 91 parallel zum Beatmungsbeutel 1 im Spontanatmungs-Bypass 9 angeordnet. Das Ventil 91 reduziert bei erhaltener Spontanatmung der zu beatmenden Person die Einatemarbeit der zu beatmenden Person. Das Spontanatmungs-Bypass-Ventil 91 ist ein Rückschlagventil, das insbesondere einen geringeren Atemwiderstand aufweist als die Kombination aus Einlassventil am Einlass 12 und das Patientenventil 15. Somit muss die zu beatmenden Person weniger Kraft und Energie aufwenden, um einen Atemhub eigenständig anzufordern. Der Schaltdruck des Rückschlagventils 91 liegt beispielsweise unterhalb 10 mbar, insbesondere unter 5 mbar, im vorliegenden Beispiel unter 2 mbar. Im Strömungskreislauf befindliches Atemgas wird der zu beatmenden Person dann über den Beatmungsbeutel 1 und über den Spontanatmungs-Bypass 9 zugeführt.

Das beschriebene Beatmungsgerät 30 stellt somit ein geschlossenes Atemsystem dar, in dem Ausatemgas wiederaufbereitet und als Einatemgas appliziert wird. Diese Wiederverwendung des Ausatemgases ermöglicht einen besonders effizienten Einsatz von zur Beatmung verwendetem Sauerstoff. Denn aufgrund der Wiederverwendung des Ausatemgases kann der bei der Atmung nicht verstoffwechselte Sauerstoff wieder zur Beatmung genutzt werden. Lediglich das verstoffwechselte Sauerstoffvolumen muss durch frisch hinzugefügten Sauerstoff ersetzt werden.

In einer Variante ist im Strömungskreislauf, bevorzugt zwischen Atemgasreservoir 5 und Frischgaszufuhr 6, ein Umschaltventil 101 vorgesehen. Dieses ermöglicht ein Umschalten von dem beschriebenen geschlossenen Atemsystem auf ein offenes Atemsystem. In einer ersten Stellung des Umschaltventils 101 ist das System wie beschrieben geschlossen und das Ausatemgas wird wiederaufbereitet als Einatemgas abgegeben. In einer zweiten Stellung des Umschaltventils 101 ist das System geöffnet, so dass die Rückatmung von Ausatemgas als Einatemgas deaktiviert ist. Dies ermöglicht eine flexiblere Handhabung des Beatmungsgeräts 30 auch in Situationen, in denen eine Beatmung mit einem geschlossenen System nicht gewünscht ist.

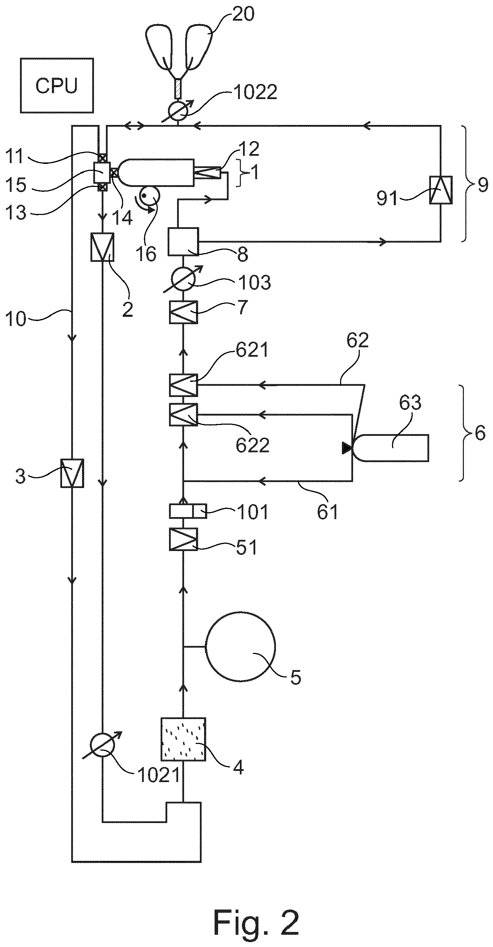

In **Figur 2** ist eine Variante des in Figur 1 dargestellten Beatmungsgeräts 30 abgebildet. Im Folgenden werden nur die Unterschiede zur Ausführungsform der Figur 1 erläutert. Die Frischgaszufuhr 6 weist zusätzlich neben der bedarfsgesteuerten Dosierung 62 mit Demandventil 621 auch eine elektronisch angesteuerte bedarfsgesteuerte Dosierung 622 auf. Auch verfügt der Beatmungsbeutel 1 über einen automatischen Antrieb 16. Mittels des automatischen Antriebs 16 wird der Beatmungsbeutel 1 komprimiert, d.h. das in ihm befindliche Atemgas ausgepresst. Somit kann das Beatmungsgerät 30 automatisch, d.h. ohne menschliche Intervention und ohne menschlichen Kraftaufwand, betrieben werden und selbsttätig Atemhübe generieren. Durch den automatischen Antrieb 16 kann eine kontrollierte maschinelle Beatmung von Personen ohne Eigenatmung erfolgen oder eine Unterstützung der selbsttätigen Atmung von Personen mit eingeschränktem eigenen Atemantrieb oder mit eingeschränkter eigener Atemkraft erfolgen oder eine Reduktion der Atemarbeit von Personen mit ausreichender Atemkraft ermöglicht werden. Vorliegend ist der automatische Antrieb 16 durch eine Vorrichtung zum maschinellen Komprimieren oder maschinellen Auspressen des Beatmungsbeutels 1 umgesetzt. Hier dargestellt ist ein Exzenter. Alternativ kann ein solcher automatischer Antrieb 16 aber auch mindestens ein Zugband, mindestens einen Kolben- oder Linearmotor, eine Vorrichtung zum pneumatischen Auspressen mittels eines Kolbens oder mittels eines Verdrängungssacks, oder eine Vorrichtung zum hydraulischen Auspressen mittels eines Kolbens oder mittels eines flüssigkeitsgefüllten Verdrängungssacks aufweisen. Ferner ist im Strömungskreislauf eine Sauerstoffmessvorrichtung 103 angeordnet. Die Sauerstoffmessvorrichtung 103 bestimmt die Sauerstoffkonzentration im wiederaufbereiteten Ausatemgas und überwacht somit die Qualität des als Einatemgas abgegebenen wiederaufbereiteten Ausatemgases. Die Sauerstoffkonzentration kann qualitativ und/oder quantitativ bestimmt werden. Die Messvorrichtung 103 weist beispielsweise einen akustischen und/oder optischen Alarm auf, der eine Abweichung der gemessenen Sauerstoffkonzentration von einem Sollwert signalisiert. Die Sauerstoffmessvorrichtung 103 ist insbesondere stromabwärts der Frischgaszufuhr 6, beispielsweise zwischen Frischgaszufuhr 6 und Beatmungsbeutel 1 bzw. Spontanatmungs-Bypass 9 angeordnet. Auch eine Kohlendioxidmessvorrichtung 102 ist im Strömungskreislauf vorgesehen. In der dargestellten Ausführungsform ist eine Kohlendioxidmessvorrichtung 1021 zur Bestimmung der Kohlendioxidkonzentration im Ausatemgas und eine Kohlendioxidmessvorrichtung 1022 zur Bestimmung der Kohlendioxidkonzentration im Einatemgas vorgesehen. Mit der ersten Kohlendioxidmessvorrichtung 1021 lässt sich auf den Kohlendioxidgehalt im Blut der zu beatmenden Person schließen, mit Hilfe der zweiten Kohlendioxidmessvorrichtung 1022 wird die Qualität des Einatemgases überwacht. In der in Fig. 2 dargestellten patientennahmen Anordnung der Kohlendioxidmessvorrichtung 1022 kann grundsätzlich auch sowohl das Einatemgas als auch das Ausatemgas mit einer einzigen Kohlendioxidmessvorrichtung 102 analysiert werden, wenn der Zeitpunkt der Messung während der Einatmung bzw. während der Ausatmung, bevorzugt am Ende der Ausatmung liegt. Die Kohlendioxidkonzentration kann qualitativ und/oder quantitativ bestimmt werden. Insbesondere die zweite Messvorrichtung 1022 kann einen akustischen und/oder optischen Alarm aufweisen, der eine Abweichung der gemessenen Kohlendioxidkonzentration von einem Sollwert signalisiert. Beispielsweise kann so eine Fehlfunktion des Atemkalks in der Extraktionseinheit 4 festgestellt werden, beispielsweise eine Erschöpfung des Atemkalks. Die Sauerstoffmessvorrichtung 103, die erste und/oder zweite Kohlendioxidmessvorrichtung 1021, 1022 sind mit einer Recheneinheit CPU des Beatmungsgeräts 30 kommunikativ verbunden. Auch der automatische Antrieb 16 und/oder die elektrisch bedarfsgesteuerte Dosierung 62 der Frischgaszufuhr 6 stehen mit der Recheneinheit in Kommunikationsverbindung. Es können auch weitere, hier nicht eingezeichnete, mit der Recheneinheit in Kommunikationsverbindung stehende Gasfluss-und/oder Drucksensoren im Strömungskreislauf vorgesehen sein, die eine Detektion und/oder Quantifizierung von selbsttätiger Atemarbeit sowie die Bestimmung der Einatmen- und Ausatem-Volumina und die Detektion von Leckagen ermöglichen. Die Kommunikationsverbindung zwischen den Messvorrichtungen 103, 1021, 1022 und der Recheneinheit CPU sowie der elektronisch angesteuerten bedarfsgesteuerten Dosierung 62 und der Recheneinheit CPU, ermöglicht eine Frischgaszufuhr in Abhängigkeit von den gemessenen Sauerstoff- und/oder Kohlendioxidkonzentrationen. Somit ist eine Steuerung und/oder Regelung der Frischgaszufuhr 6 basierend auf einer gemessenen Sauerstoff- und/oder Kohlendioxidkonzentration möglich. Durch die Kommunikationsverbindung zwischen den (nicht dargestellten) Druck- und/oder Gasflusssensoren, insbesondere auch den Messvorrichtungen 103, 1021, 1022, und dem automatischen Antrieb 16 mit der Recheneinheit CPU, wird eine Steuerung und/oder Regelung des automatischen Antriebs 16 in Abhängigkeit von den gemessenen Sauerstoff- und/oder Kohlendioxidkonzentrationen und/oder in Abhängigkeit von den gemessenen Drücken und/oder Gasflüssen und/oder Atemzug- bzw. Atemhub-Volumina ermöglicht. Insbesondere können so die maschinellen Atemhübe mit der selbsttätigen Atemarbeit einer zu beatmenden Person synchronisiert werden. Die Druck- und/oder Gasflusssensoren sind beispielsweise patientenseitig am Patientenanschluss, insbesondere patientennah, positioniert.

Insbesondere wenn anstelle der Kombination von Konstantdosierung 61 und bedarfsgesteuerter Dosierung 62 nur eine der beiden Dosierungen verwendet wird, sollte eine engmaschige, insbesondere kontinuierliche Überwachung erfolgen. Bei alleiniger Verwendung der Konstantdosierung 61 sollte der Füllzustand des Atemgasreservoirs 5 sowie die Sauerstoffkonzentration überwacht werden. Die Konstantdosierung 61 sollte dann engmaschig oder kontinuierlich an den aktuellen Füllzustand und die aktuelle Sauerstoffkonzentration angepasst werden. Bei alleiniger Verwendung der bedarfsgesteuerten Dosierung 62 sollte die Sauerstoffkonzentration überwacht werden. Die bedarfsgesteuerte Dosierung 62 sollte dann engmaschig oder kontinuierlich an die aktuelle Sauerstoffkonzentration angepasst werden. Eine Kontrolle des Füllzustands ist in diesem Fall nicht notwendig, da das Beatmungsgerät 30 bei bedarfsgesteuerter Dosierung 62 über eine selbsttätige intrinsische Volumenregelung zur Aufrechterhaltung des Gasvolumens verfügt.

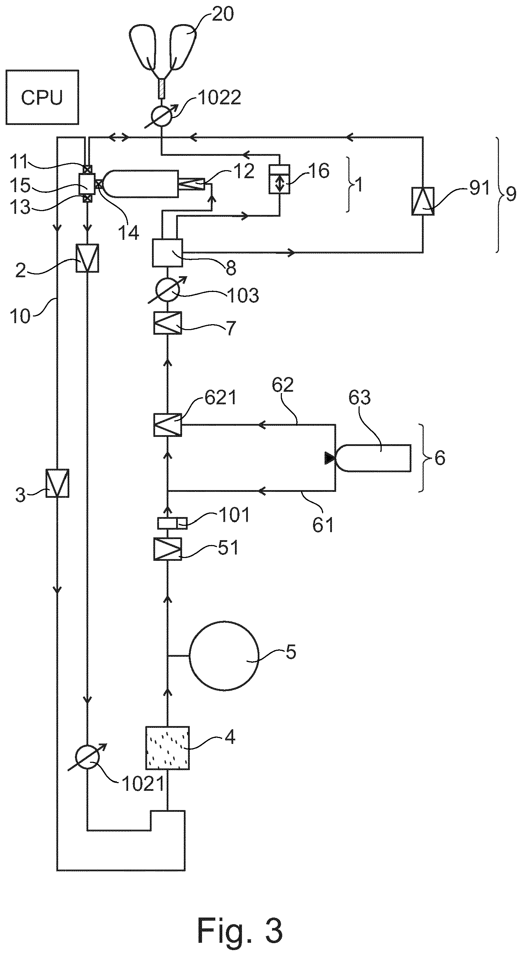

In **Figur 3** ist eine alternative Ausführungsform des Beatmungsgeräts 30 dargestellt. Sie unterscheidet sich von der Ausführungsform der Figur 2 in der Art des automatischen Antriebs 16. Während im Ausführungsbeispiel der Figur 2 eine Vorrichtung zum maschinellen Auspressen, beispielsweise ein Exzenter, verwandt wird, kommt vorliegend eine parallel zum Beatmungsbeutel 1 geschaltete Pumpe als automatischer Antrieb 16 zum Einsatz. Als parallel geschaltete Pumpe kann beispielsweise ein Kolbenpumpe, eine Rotationspumpe, eine Flügelpumpe, eine Turbine oder eine Membranpumpe zum Einsatz kommen. Es ist auch denkbar, dass mehrere Pumpen in dem parallel zum Beatmungsbeutel 1 geführten Arm des Strömungskreislaufs angeordnet sind. Auch hier kann eine Recheneinheit CPU zur Steuerung und/oder Regelung wie im Zusammenhang mit Figur 2 beschrieben zum Einsatz kommen.

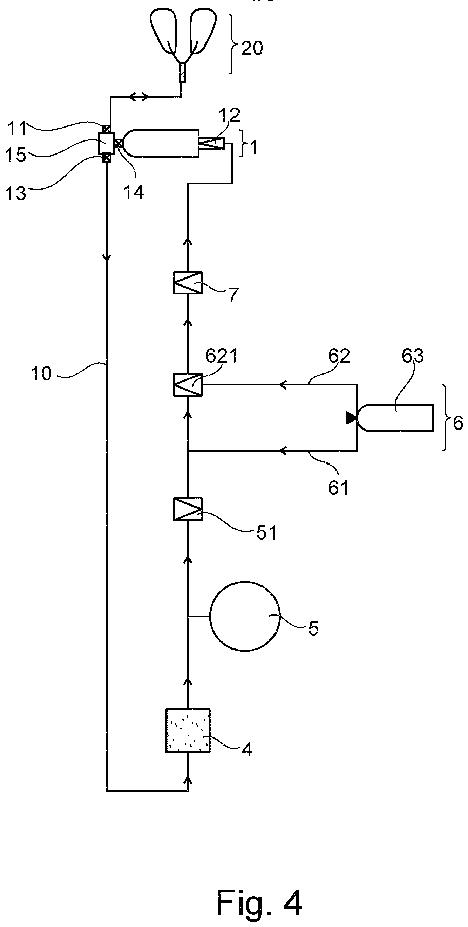

In **Figur 4** ist eine weitere Ausführungsform des Beatmungsgeräts 30 dargestellt. Die dargestellte Ausführungsform weist eine minimale Anzahl an Komponenten auf. Sie ist somit im Vergleich zu der in der Figur 1 dargestellten Ausführungsform weniger komplex und kostengünstiger herzustellen. Im Folgenden werden lediglich die Abwandlungen zu der in Figur 1 dargestellten Ausführungsform beschrieben. Im Gegensatz zur Ausführungsform der Figur 1, weist das dargestellte Beatmungsgerät 30 lediglich einen Beatmungsbeutel 1 auf. Der Beatmungsbeutel 1, d.h. dessen Ausatemgas-Auslass 13, ist mit der Extraktionseinheit 4 verbunden. Ferner ist stromabwärts der Extraktionseinheit 4 ein Atemgasreservoir 5 vorgesehen, das ein Überdruckventil 51 zum Ablassen von Atemgas an die Umgebung aufweist. Stromabwärts ist eine Frischgaszufuhr 6, beispielsweise mit Konstantdosierung 61 und bedarfsgesteuerter Dosierung 62, hier mittels Demandventil 621, im Strömungskreislauf angeordnet. Im Strömungskreislauf istferner ein Noteinlassventil 7, beispielsweise stromabwärts der Frischgaszufuhr 6, angeordnet. Ein ABC-Filter 8 kann optional vorgesehen sein. Das in der Extraktionseinheit 4 gereinigte Ausatemgas wird mittels der Frischgaszufuhr 6 durch Zufuhr von Sauerstoff wiederaufbereitet und durch ein Einlassventil 12 in den Beatmungsbeutel 1 gesogen. Die Applikation des wiederaufbereiteten Ausatemgases erfolgt über einen Patientenanschluss 11 des Beatmungsbeutels. Ein Umschaltventil 101 kann optional vorgesehen sein.

Im Gegensatz zur Ausführungsform der Figur 1 wird vorliegend auf einen Spontanatmungs-Bypass 9 und darin angeordnetes Rückschlagventil 91 verzichtet. Die dargestellte Variante ermöglicht somit keine Spontanatmung mit reduziertem Einatemwiderstand, sondern nur eine Spontanatmung durch den Beatmungsbeutel 1 hindurch mit höherem Atemwiderstand infolge des Einlassventils 12 und Zwei-WegeVentils 15. Ein PEEP-Ventil 2 kann optional vorgesehen sein. Bei Verwendung ohne PEEP-Ventil 2 kann stromabwärts des Ausatemgas-Auslasses 13 ein Rückschlagventil positioniert sein, um insbesondere bei Spontanatmung eine Rückatmung von Ausatemgas entgegen der Soll-Strömungsrichtung zu verhindern. Ferner ist ein Atemwegs-Überdruckventil 3 nur optional vorgesehen. Es erfolgt keine Rückführung von Atemgas durch den Patientenanschluss 11 über ein Atemwegs-Überdruckventil 3 in den Strömungskreislauf. Das Atemgas wird bei einem Ansprechen des Atemwegs-Überdruckventil 3 in die Umgebung abgegeben und nicht in den Strömungskreislauf zurückgeführt.

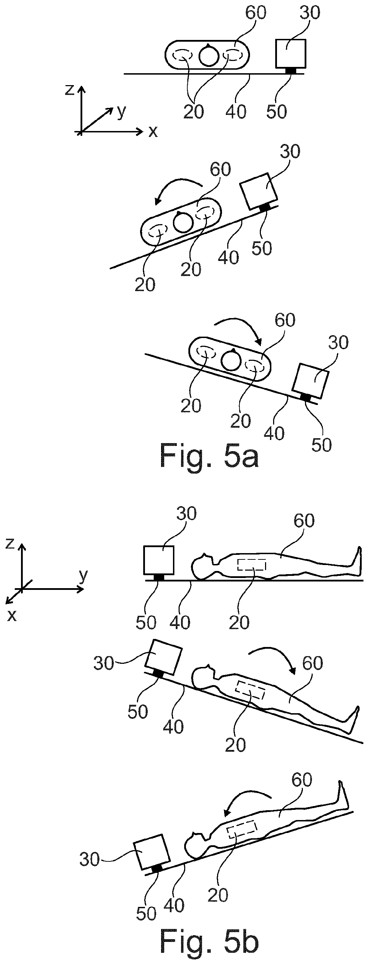

**Figuren 5a und 5b** illustrieren die Verwendung eines Beatmungsgeräts 30 gemäß einer Ausführungsform. Die dargestellte Verwendung ermöglicht eine Beatmung einer Person 60 unter Wasser, ohne dass hierzu eine manuelle oder angetriebene Kompression des Beatmungsbeutels 1 notwendig ist. Das Beatmungsgerät 30 ist zur Verwendung unter Wasser mit einer Patiententrage 40 verbunden. Hierzu weist das Beatmungsgerät beispielsweise ein Befestigungselement 50 auf. Die zu beatmende Person 60 ist ebenfalls mit der Patiententrage 40 verbunden. Anstelle einer Patiententrage 40 kann auch jedes andere geeignete Befestigungsgestell zur relativen Fixierung von zu beatmender Person 60 und Beatmungsgerät 30 verwendet werden. Das Befestigungselement 50 weist beispielsweise Schraub- und/oder Klemmverbindungen und/oder Ösen und/oder Schlaufen und/oder Gurte auf. Das Befestigungselement 50 garantiert eine in den drei Raumrichtungen definierte Position des Beatmungsgerätes 30 relativ zur zu beatmenden Person 60. Das Beatmungsgerät 30 ist ungefähr auf Höhe der Lunge 20 der zu beatmenden Person 60 angeordnet.

In der in Figur 5a dargestellten Ausführungsform ist das Beatmungsgerät 30 entlang der y-Achse ungefähr auf Höhe der Lungenmitte an der Patiententrage 40 befestigt. Entlang der x-Achse ist das Beatmungsgerät 30 relativ zur zu beatmenden Person 60 einseitig außerhalb der Lungenmitte positioniert, insbesondere links oder rechts der Lungenmitte. In der in Figur 5b dargestellten Ausführungsform ist das Beatmungsgerät 30 entlang der x-Achse ungefähr auf Höhe der Lungenmitte angeordnet, während das Beatmungsgerät 30 entlang der y-Achse einseitig außerhalb der Lungenmitte positioniert ist, insbesondere kopf- oder fußseitig der Lungenmitte. Dabei entspricht die y-Achse einer Längsachse einer zu beatmenden Person und die x-Achse einer in der Ebene der Person liegende, zur Längsachse senkrechte Querachse.

Für den Einsatz unter Wasser sind die Verbindungselemente 10 des Beatmungsgeräts 30, soweit vorhanden, fest fixiert und die auftriebsreichen Bauteile, insbesondere der Beatmungsbeutel 1 und das Atemgasreservoir 5 durch direkte Befestigung und/oder durch eine Positionierung in auftriebsbegrenzenden Elementen, beispielsweise Rohren, vor unbeabsichtigtem Aufschwimmen geschützt.

Bedingt durch die hydrostatische Druckdifferenz beim Einsatz unter Wasser von 1mbar pro Zentimeter Höhendifferenz kann durch eine Veränderung der hydrostatischen Lage des Beatmungsgerätes 30 in Bezug zur zu beatmenden Person 60 eine Ein- oder Ausatmung erzielt werden, ohne dass hierfür ein Auspressen des Beatmungsbeutels 1 erforderlich wäre. Wird das Beatmungsgerät 30 oberhalb der zu beatmenden Person 60 - also in einer geringeren Wassertiefe - positioniert, so strömt das Atemgas aus der zu beatmenden Person 60 heraus und in das Beatmungsgerät 30 hinein. Somit kann eine Ausatmung erzielt werden. Wird das Beatmungsgerät 30 unterhalb der zu beatmenden Person 60 - also in einer größeren Wassertiefe - positioniert, so strömt Atemgas aus dem Beatmungsgerät 30 in die zu beatmende Person 60 hinein. Somit kann eine Einatmung erzielt werden. Durch gezieltes Positionieren des Beatmungsgerätes 30 um x cm tiefer als die Lungenmitte der zu beatmenden Person kann somit gezielt ein definierter Beatmungsdruck von x mbar erzeugt werden und somit ein entsprechendes Atemzugvolumen appliziert werden. Eine Höhendifferenz von 20 cm und eine entsprechende hydrostatische Druckdifferenz von 20 cm Wassersäule erzeugt einen definierten Beatmungsdruck von 20 mbar im Süßwasser. Im Salzwasser werden geringfügig höhere Beatmungsdrücke erzielt. Die Höhe des gewünschten Beatmungsdruckes und somit auch des gewünschten Atemzugvolumens kann somit gezielt durch die hydrostatische Druckdifferenz zwischen Beatmungsgerät 30 und Lungenmitte der zu beatmenden Person 60 bestimmt werden.

Die gewünschte hydrostatische Druckdifferenz kann entweder durch ein Drehen der zu beatmenden Person 60 um die Körperachse, hier y-Achse, erzielt werden. Die Drehung erfolgt abwechselnd im und gegen den Uhrzeigersinn, siehe Figur 5a. Alternativ kann die gewünschte hydrostatische Druckdifferenz durch ein Kippen in Richtung der Körperachse, hier x-Achse, erzielt werden. Hierbei wird zwischen einer Kopf-hoch-Lagerung und einer Kopf-tief-Lagerung gewechselt, siehe Figur 5b.

Ein im Beatmungsgerät 30 unter Umständen verbautes Atemwegs-Überdruckventil 3 ist bei dieser Verwendung nicht voll funktionsfähig. Denn bei Überschreiten des Schaltdrucks des Atemwegs-Überdruckventils 3 müsste zusätzlich die hydrostatische Druckdifferenz zwischen Beatmungsgerät 30 und Lungenmitte der zu beatmeten Person 60 überwunden werden. Für die Beatmung unter Wasser mittels Dreh- oder Kippbewegungen sollte daher mindestens ein Atemwegs-Überdruckventil 3 auf Höhe der Lungenmitte der zu beatmenden Person 60 angeordnet sein, welches beim Öffnen infolge des Erreichens oder Überschreitens des Schaltdrucks eine Abgabe von Atemgas ins Wasser ermöglicht. Zur Vermeidung von Gasblasen sollten hier bei militärischen Einsätzen zur Vermeidung einer Enttarnung mindestens ein Blasendiffusor und/oder ein Sauerstoffreduktor mit dem Auslass des Atemwegs-Überdruckventils 3 verbunden oder in diesen integriert sein.

### Bezugszeichenliste

- 1: Beatmungsbeutel
- 11: Patientenanschluss
- 12: Einlassventil Beatmungsbeutel
- 13: Ausatemgas-Auslass Beatmungsbeutel
- 14: Einatemgas-Auslass Beatmungsbeutel
- 15: Zwei-Wege-Ventil
- 16: automatischer Antrieb
- 2: PEEP-Ventil
- 3: Atemwegs-Überdruckventil
- 4: Extraktionseinheit
- 5: Atemgasreservoir
- 51: Atemgasreservoir-Überdruckventil
- 6: Frischgaszufuhr
- 61: Konstantdosierung
- 62: bedarfsgesteuerte Dosierung
- 621: Demandventil
- 622: elektronisch gesteuertes Ventil
- 63: Sauerstoffquelle
- 7: Noteinlassventil
- 8: ABC-Filter
- 9: Spontanatmungs-Bypass
- 91: Spontanatmungs-Bypass-Ventil
- 10: Verbindungselement
- 101: Umschaltventil
- 1021: Kohlendioxidmessvorrichtung
- 1022: Kohlendioxidmessvorrichtung
- 103: Sauerstoffmessvorrichtung
- 20: Lunge
- 30: Beatmungsgerät
- 40: Patiententrage
- 50: Befestigungselement
- 60: zu beatmende Person
- x: erste Achse
- y: zweite Achse
- z: dritte Achse

## Patentansprüche

1. Beatmungsgerät (30) zur Beatmung einer Person, aufweisend einen Strömungskreislauf für Atemgas mit:
- einem selbstinflatierenden Beatmungsbeutel (1), wobei der Beatmungsbeutel (1) einen Patientenanschluss (11) aufweist und wobei der Beatmungsbeutel (1) weiter einen Auslass (13) zur Einleitung eines Ausatemgases in den Strömungskreislauf und einen Einlass (12) zum Ansaugen eines wiederaufbereiteten Ausatemgases aus dem Strömungskreislauf in den Beatmungsbeutel (1) aufweist;
- einer Extraktionseinheit (4) zum Entfernen von Kohlendioxid aus dem Ausatemgas;
- einer Frischgaszufuhr (6) zur Zufuhr von Sauerstoff in den Strömungskreislauf; und
- einem Atemgasreservoir (5) zur Zwischenspeicherung von im Strömungskreislauf befindlichem Atemgas.

2. Beatmungsgerät (30) nach einem der vorhergehenden Ansprüche, wobei der Strömungskreislauf mindestens ein Verbindungselement (10) zur Verbindung von im Strömungskreislauf angeordneten Komponenten des Beatmungsgeräts (30) aufweist.

3. Beatmungsgerät (30) nach einem der vorhergehenden Ansprüche, wobei ein Ventil (2) zur Erzeugung eines positiven endexspiratorischen Drucks (PEEP), insbesondere zwischen dem Auslass (13) des Beatmungsbeutels (1) und dem Eingang der Extraktionseinheit (4), angeordnet ist.

4. Beatmungsgerät (30) nach einem der Ansprüche 1 bis 2, wobei durch eine Verringerung eines Strömungsquerschnitts der Extraktionseinheit (4) und/oder der Verbindungselemente (10) in Strömungsrichtung die Erzeugung eines positiven endexspiratorischen Drucks (PEEP) ermöglicht wird.

5. Beatmungsgerät (30) nach einem der vorhergehenden Ansprüche, wobei ein Atemwegs-Überdruckventil (3) zum Ablassen von Atemgas aus dem Beatmungsbeutel (1) und zur Zuführung des abgelassenen Atemgases an die Extraktionseinheit (4) im Strömungskreislauf, insbesondere zwischen dem Patientenanschluss (11) und dem Eingang der Extraktionseinheit (4), angeordnet ist.

6. Beatmungsgerät (30) nach einem der vorhergehenden Ansprüche, wobei das Atemgasreservoir (5) ein Atemgasreservoir-Überdruckventil (51) aufweist, welches das Ablassen von überschüssigem Atemgas in die Umgebung ermöglicht.

7. Beatmungsgerät (30) nach einem der vorherigen Ansprüche, wobei die Frischgaszufuhr (6) stromabwärts hinter dem Atemgasreservoir (5) angeordnet ist.

8. Beatmungsgerät (30) nach einem der vorherigen Ansprüche, wobei ein Noteinlassventil (7) zum Einlass von Umgebungsluft in den Strömungskreislauf, insbesondere zwischen der Frischgaszufuhr (6) und dem Einlass (12) des Beatmungsbeutels (1), im Strömungskreislauf angeordnet ist.

9. Beatmungsgerät (30) nach einem der vorhergehenden Ansprüche, wobei ein Filter (8) zur Entfernung von toxischen Gasen und/oder toxischen Partikeln aus dem Strömungskreislauf, insbesondere zwischen der Frischgaszufuhr (6) und dem Einlass (12) des Beatmungsbeutels (2), im Strömungskreislauf angeordnet ist.

10. Beatmungsgerät (30) nach einem der vorhergehenden Ansprüche, wobei die Frischgaszufuhr (6) eine Sauerstoffquelle (63) oder einen Anschluss für eine externe Sauerstoffquelle aufweist.

11. Beatmungsgerät (30) nach einem der vorhergehenden Ansprüche, wobei die Frischgaszufuhr (6) eine bedarfsgesteuerte Dosierung (62) und/oder eine Konstantdosierung (61) aufweist.

12. Beatmungsgerät (30) nach einem der vorhergehenden Ansprüche, wobei ein Spontanatmungs-Bypass (9) mit Rückschlagventil (91), insbesondere zwischen Frischgaszufuhr (6) und einem Auslass des Strömungskreislaufes, parallel zum Beatmungsbeutel (1) angeordnet ist, wobei der Auslass geeignet ist, mit der Lunge (20) einer an das Beatmungsgerät (30) angeschlossenen Person verbunden zu werden.

13. Beatmungsgerät (30) nach einem der vorhergehenden Ansprüche, wobei im Strömungskreislauf eine Sauerstoffmessvorrichtung (103) zur Bestimmung einer Sauerstoffkonzentration im wiederaufbereiteten Ausatemgas angeordnet ist.

14. Beatmungsgerät (30) nach einem der vorhergehenden Ansprüche, wobei im Strömungskreislauf eine Kohlendioxidmessvorrichtung (1021) zur Bestimmung einer Kohlendioxidkonzentration im Ausatemgas und/oder eine Kohlendioxidmessvorrichtung (1022) zur Bestimmung einer Kohlendioxidkonzentration im wiederaufbereiteten Ausatemgas angeordnet ist.

15. Beatmungsgerät (30) nach einem der vorhergehenden Ansprüche, wobei das Beatmungsgerät (30) einen automatischen Antrieb (16) zur Kompression des Beatmungsbeutels (1) aufweist.

16. Beatmungsgerät (30) nach Anspruch 15, wobei das Beatmungsgerät (30) eine Recheneinheit (CPU) zur Steuerung und/oder Regelung des automatischen Antriebs (16) aufweist.

17. Beatmungsgerät (30) nach einem der vorhergehenden Ansprüche, wobei das Beatmungsgerät (30) ein Befestigungselement (50) zur Befestigung des Beatmungsgeräts (30) an einer Patiententrage (40) aufweist.

18. Verfahren zur Wiederaufbereitung von Ausatemgas in einem Strömungskreislauf eines Beatmungsgeräts (30), mit den Schritten:
- Einleitung von Ausatemgas in einen in dem Strömungskreislauf angeordneten selbstinflatierenden Beatmungsbeutel (1) über einen Patientenanschluss (11) des Beatmungsbeutels (1) durch einen Auslass (13) des Beatmungsbeutels (1) in den Strömungskreislauf;
- Entfernen von Kohlendioxid aus dem Ausatemgas mittels einer im Strömungskreislauf angeordneten Extraktionseinheit (4);
- Zwischenspeicherung von im Strömungskreislauf befindlichem Atemgas in einem Atemgas-Reservoir (5) des Beatmungsgeräts (30);
- Zufuhr von Sauerstoff in den Strömungskreislauf mittels einer Frischgaszufuhr (6);
- Ansaugen eines wiederaufbereiteten Ausatemgases durch einen Einlass (12) des Beatmungsbeutels (1) in den Beatmungsbeutel (1) durch Dekompression des Beatmungsbeutels (1); und
- Ausstoß des wiederaufbereiteten Ausatemgases aus dem Strömungskreislauf durch den Patientenanschluss (11) des Beatmungsbeutels (1) durch Kompression des Beatmungsbeutels (1).

19. Verfahren nach Anspruch 18, wobei während der Einleitung des Ausatemgases in den Strömungskreislauf ein positiver exspiratorischer Druck (PEEP) in der Lunge (20) aufrechterhalten wird.

20. Verfahren nach einem der vorhergehenden Ansprüche 18 bis 19, wobei bei Überschreitung eines definierten Drucks des Atemgases in dem Beatmungsbeutel (1) Atemgas aus dem Beatmungsbeutel (1) mittels eines Atemwegs-Überdruckventils (3) abgelassen wird und der Extraktionseinheit (4) zugeführt wird.

21. Verfahren nach einem der vorhergehenden Ansprüche 18 bis 20, wobei die Zufuhr von Sauerstoff über eine Frischgaszufuhr (6) mit Konstantdosierung (61) und/oder über eine Frischgaszufuhr (6) mit bedarfsgesteuerter Dosierung (62) erfolgt.

22. Verfahren nach Anspruch 21, wobei die Konstantdosierung (61) deaktiviert wird und ausschließlich eine bedarfsgesteuerte Dosierung (62) der Frischgaszufuhr (6) erfolgt.

23. Verfahren nach einem der vorhergehenden Ansprüche 18 bis 22, wobei die Abgabe des wiederaufbereiteten Ausatemgases aus dem Strömungskreislauf zusätzlich durch einen Spontanatmungs-Bypass (9) erfolgt, wobei der Spontanatmungs-Bypass (9) ein parallel zum Beatmungsbeutel (1) angeordnetes Rückschlagventil (91) aufweist.

24. Verfahren nach einem der vorhergehenden Ansprüche 18 bis 23, wobei mittels mindestens eines im Strömungskreislauf angeordneten Filters (8) toxische Gase und/oder toxische Partikel aus dem Strömungskreislauf entfernt werden.

25. Verfahren nach einem der vorhergehenden Ansprüche 18 bis 24, wobei eine Sauerstoffkonzentration des wiederaufbereiteten Ausatemgases gemessen wird.

26. Verfahren nach einem der vorhergehenden Ansprüche 18 bis 25, wobei die Kohlendioxidkonzentration des Ausatemgases und/oder die Kohlendioxidkonzentration des wiederaufbereiteten Ausatemgases gemessen wird.

27. Verfahren nach einem der vorhergehenden Ansprüche 18 bis 26, wobei die Kompression des Beatmungsbeutels durch einen automatischen Antrieb erfolgt.

28. Verwendung eines Beatmungsgeräts (30) nach einem der Ansprüche 1 bis 17 zur Beatmung einer Person.
